# EUROPEAN PATENT APPLICATION

(11) **EP 3 406 221 A1**
(43) Date of publication of application: **28.11.2018**
(21) Application number: 16886342.1
(22) Date of filing: 22.01.2016
(51) Int. Cl.: A61B 34/30, A61B 90/00, B25J 9/06

(54) **MEDICAL MANIPULATOR SYSTEM**

(71) Applicant: OLYMPUS CORPORATION, Hachioji-shi Tokyo 192-8507 (JP)
(72) Inventor: HASEGAWA, Mitsuaki, Tokyo 192-8507 (JP); ISODA, Takumi, Tokyo 192-8507 (JP)
(74) Representative: Schicker, Silvia
(86) International application number: PCT/JP2016/051833
(87) International publication number: WO 2017/126101

(57) **Abstract**

A control unit (100) of a medical manipulator system has a position recognition unit (101) that recognizes the positions of a first manipulator and a second manipulator, an interference prediction unit (103) that predicts whether or not the first manipulator interferes with the second manipulator in a case where the first manipulator is moved along a first movement path, and a mode selection unit (107) that selects any one of a first mode and a second mode such that the first mode is selected in a case where the interference prediction unit (103) predicts that the first manipulator does not interfere with the second manipulator, and the second mode is selected in a case where the interference prediction unit (103) predicts that the first manipulator interferes with the second manipulator, and selects one mode from a plurality of operation modes so as to shift from the first mode or the second mode to a third mode in a case where a torque sensor (14) detects contact between the first manipulator and an obstacle while the control unit (100) is operating in the first mode or the second mode.

## Description

### Technical Field

The present invention relates to a medical manipulator system.

### Background Art

Medical manipulator systems that perform a surgical operation by operating a plurality of arms have been known hitherto. For example, Patent Document 1 discloses a medical manipulator system that has a plurality of arms capable of mounting a surgical tool and that can move the plurality of arms such that the surgical tool is swung with an incision position for introducing the surgical tool into the body as a center (remote center).

The medical manipulator system disclosed in Patent Document 1 can automatically or manually move the arms while maintaining the position of the remote center.

### Citation List

### Patent Literature

[Patent Document 1] United States Patent Application, Publication No. 2013/325031

### Summary of Invention

### Technical Problem

In a case in which the plurality of arms of the medical manipulator system interfere with each other, an operator or the like of the medical manipulator system may temporarily halt a treatment in order to manually eliminate any interference between arms. In order to make it hard to cause the halt of the treatment due to the interference between the arms, it is considered that the arms are automatically operated such that the interference between the arms is avoided by predicting the interference. However, in the manipulator described in Patent Document 1, obstacles (an operator, an assistant, a patient, other medical instruments, or the like) located around a manipulator cannot be recognized. Thus, if the arms are automatically operated so as to avoid the interference between the arms, there is a possibility that the arms may collide against the obstacle in a case in which the obstacle is on a movement path of the arms.

The invention has been made in view of the above-described circumstances, and an object thereof is to provide a medical manipulator system that can rapidly avoid any interference between arms even if an obstacle is on a movement path for avoiding the interference between arms.

### Solution to Problem

One aspect of the invention is a medical manipulator system including a first manipulator having a joint group; a first manipulation unit that issues a command for manipulating the first manipulator; a control unit that receives the command and controls the first manipulator according to one operation mode among a plurality of operation modes; a contact detection unit that detects that the first manipulator and an obstacle have come into contact with each other; and a second manipulator capable of operating independently from or in cooperation with the first manipulator. The joint group includes one or more joints belonging to a first group, and one or more joints belonging to a second group. The plurality of operation modes includes a first mode in which the first manipulator is controlled such that the first manipulator moves along a first movement path on which the first manipulator is operated only using the joints belonging to the first group, a second mode in which the first manipulator is controlled such that the first manipulator moves along the second movement path on which the first manipulator is moved using the joints belonging to the first group and the joints belonging to the second group, and a third mode in which the first manipulator is operated so as to cancel a contact state between the first manipulator and the obstacle when the contact detection unit detects the contact. The control unit includes a position recognition unit that recognizes positions of the first manipulator and the second manipulator, an interference prediction unit that predicts whether or not the first manipulator interferes with the second manipulator in a case in which the first manipulator is moved along the first movement path, and a mode selection unit that selects any one of the first mode and the second mode such that first mode is selected in a case in which the interference prediction unit predicts that the first manipulator does not interfere with the second manipulator, and second mode is selected in a case in which the interference prediction unit predicts that the first manipulator interferes with the second manipulator, and selects one mode from plurality of operation modes so as to shift from the first mode or the second mode to the third mode in a case in which the contact detection unit detects the contact between the first manipulator and the obstacle while the control unit is operating in the first mode or the second mode. The control unit sets a path for detouring the position of the second manipulator as the second movement path in the second mode.

The joints included in the second group may include a redundant joint that has a redundant relationship with the joints included in the first group.

The control unit may include, as a control procedure of the first manipulator in the third mode, a command stop step of stopping the control of the first manipulator based on the command, a movement amount calculation step of calculating a movement amount of the first manipulator for canceling a contact state with the obstacle, a movement step of moving the first manipulator by the movement amount calculated in the movement amount calculation step, and a return step of shifting the third mode to a mode which is a mode before shift to the third mode and is one of the first mode and the second mode. The control unit may control the first manipulator according to the control procedure of the first manipulator in the third mode.

The first manipulator may further include a second manipulation unit that is capable of operating the first manipulator and is different from the first manipulation unit. The control unit includes, as a control procedure of the first manipulator in the third mode, a command stop step of stopping the control of the first manipulator based on the command, and a permission step of permitting the second manipulation unit to manipulate the first manipulator. The control unit may control the first manipulator according to the control procedure of the first manipulator in the third mode.

The second manipulation unit may further include an end detection unit that detects end of the manipulation performed by the second manipulation unit. The control unit may further include, as a control procedure of the first manipulator in the third mode, a return step of shifting the third mode to a mode which is a mode before shift to the third mode in a case in which the end detection unit detects the end of the manipulation performed by the second manipulation unit. The control unit may control first manipulator according to the control procedure of the first manipulator in the third mode.

### Advantageous Effects of Invention

According to the invention, it is possible to provide the medical manipulator system that can rapidly avoid any interference between arms even if the obstacle is on the movement path for avoiding the interference between arms.

### Brief Description of Drawings

FIG. 1 is a schematic view illustrating a medical manipulator system of a first embodiment of the invention.
FIG. 2 is a schematic view illustrating a first manipulator of the medical manipulator system.
FIG. 3 is a block diagram illustrating main units of the medical manipulator system.
FIG. 4 is a flowchart illustrating a control procedure in a control unit of the medical manipulator system.
FIG. 5 is a flowchart illustrating the flow of a manipulation using the medical manipulator system.
FIG. 6 is a view for explaining the working of the medical manipulator system.
FIG. 7 is a view for explaining the working of the medical manipulator system.
FIG. 8 is a view for explaining the working of the medical manipulator system.
FIG. 9 is a schematic view illustrating the first manipulator in a modification example of the medical manipulator system of the embodiment.
FIG. 10 is a schematic view illustrating a medical manipulator system of a second embodiment of the invention.
FIG. 11 is a view for explaining the working of the medical manipulator system.
FIG. 12 is a block diagram illustrating main units of a medical manipulator system of a third embodiment of the invention.
FIG. 13 is a view for explaining the working of the medical manipulator system.
FIG. 14 is a view for explaining the working of the medical manipulator system.
FIG. 15 is a block diagram illustrating main units of a medical manipulator system of a fourth embodiment of the invention.
FIG. 16 is a flowchart illustrating the flow of a manipulation using the medical manipulator system.
FIG. 17 is a schematic view illustrating the first manipulator in a modification example of the medical manipulator system of the embodiment.

### Description of Embodiments

### (First Embodiment)

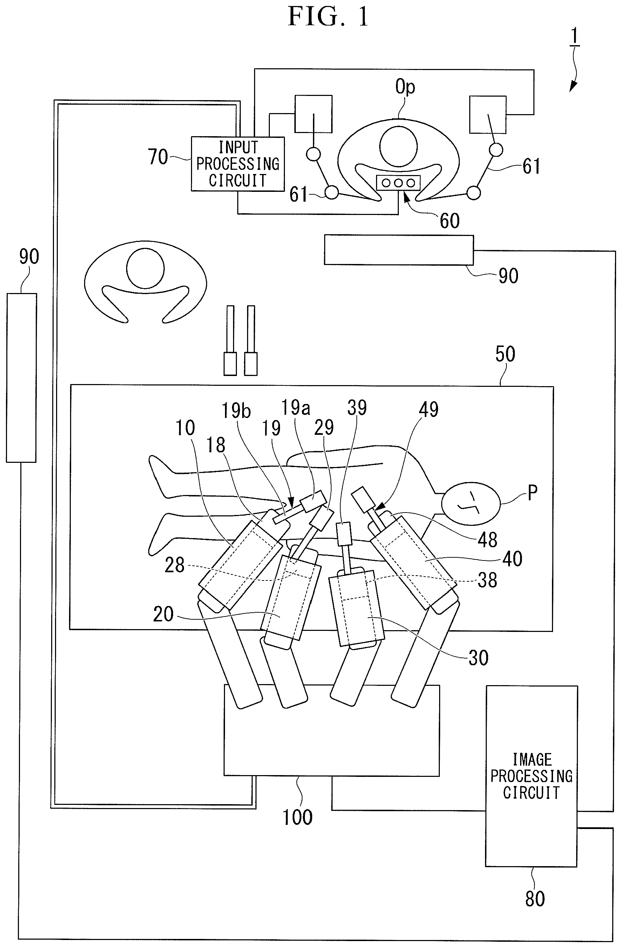

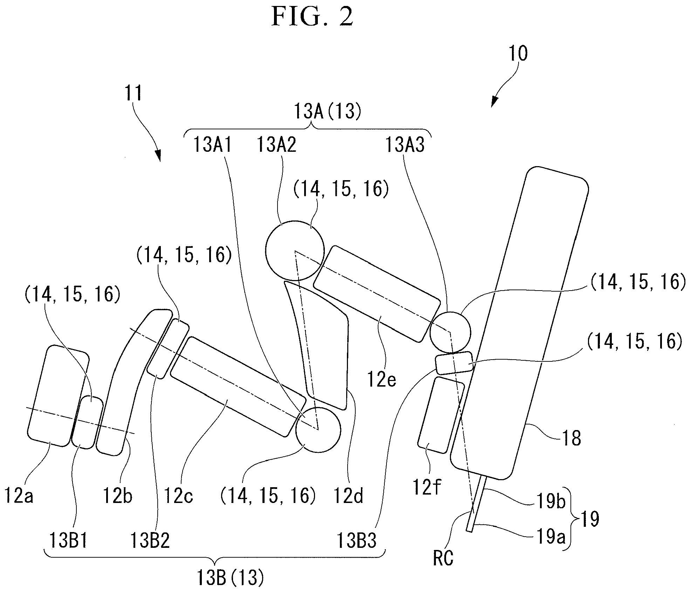

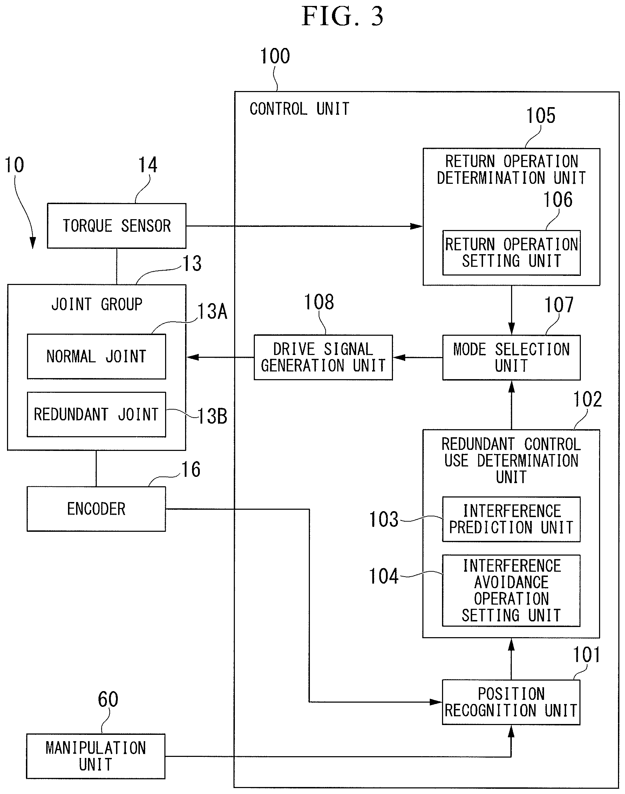

A first embodiment of the invention will be described. FIG. 1 is a schematic view illustrating a medical manipulator system of the present embodiment. FIG. 2 is a schematic view illustrating a first manipulator of the medical manipulator system. FIG. 3 is a block diagram illustrating main units of the medical manipulator system.

A medical manipulator system 1 of the present embodiment illustrated in FIG. 1 is a master-slave type manipulator system. The medical manipulator system 1 has a plurality of slave manipulators (a first manipulator 10, a second manipulator 20, a third manipulator 30, a fourth manipulator 40), a surgical bed 50, a manipulation unit 60, an input processing circuit 70, an image processing circuit 80, a display unit 90, and a control unit 100.

The plurality of slave manipulators 10, 20, 30, and 40 are installed in the vicinity of the surgical bed 50 on which a patient P is placed. In addition, the slave manipulators 10, 20, 30, and 40 may be installed in the surgical bed 50.

In the following, the configuration of the first manipulator 10 will be described in detail. Since the second manipulator 20, the third manipulator 30, and the fourth manipulator 40 have the same configuration as that of the first manipulator 10, the detailed description hereof will be omitted.

As illustrated in FIG. 2, the first manipulator 10 has an arm 11 and an adapter 18 for attaching a surgical tool 19.

The arm 11 has a link group 12, a joint group 13, a torque sensor 14, an actuator 15, and an encoder 16.

The link group 12 has a plurality of links 12a, 12b, 12c, 12d, 12e, and 12f. The plurality of links 12a, 12b, 12c, 12d, 12e, and 12f are movable by respective joints (to be described below) included in the joint group 13 with pivot axes peculiar to the joints as pivot centers. The adapter 18 for attaching the surgical tool 19 is disposed on the link 12f located closest to a distal side among the plurality of links 12a, 12b, 12c, 12d, 12e, and 12f.

The joint group 13 has joints 13A1, 13A2, and 13A3 belonging to a first group (normal joint 13A), and joints 13B1, 13B2, and 13B3 belonging to a second group (redundant joint 13B).

The joint 13A1, 13A2, and 13A3, which are included in the normal joint 13A, in the joint group 13 of the first manipulator 10 become, for example, driving shafts for at least one of yawing, pitching, and rolling and are preferentially used during the operation of the first manipulator 10. The number of joints included in the normal joint 13A may be appropriately selected in consideration of the degrees of freedom required for the first manipulator 10.

The joints 13B1, 13B2, and 13B3, which are included in the redundant joint 13B, in the joint group 13 of the first manipulator 10, have a redundant relationship with respect to the above normal joint (the joints 13A1, 13A2, and 13A3), respectively. The redundant joint 13B gives more redundant degrees of freedom than the degrees of freedom that the normal joint 13A gives to the first manipulator 10 to the first manipulator 10. In the present embodiment, the redundant joint 13B is allocated to all the joints belonging to the normal joint 13A.

The torque sensor 14 is a sensor that detects the magnitude of the torque applied each joint that constitutes the joint group 13. The torque sensor 14 is connected to the control unit 100.

The actuator 15 operates the joint group 13. The actuator 15 is able to operate only the joints included in the normal joint 13A or operate both the normal joint 13A and the redundant joint 13B in accordance with a control using the control unit 100.

The encoder 16 detects the amount of operation of the joint group 13. The encoder 16 is connected to the control unit 100. Accordingly, the control unit 100 is able to recognize the amount of operation of the joint group 13.

The surgical tool 19 attached to the first manipulator 10 includes an end effector 19a at a distal end of an elongated shaft 19b. The end effector 19a of the surgical tool 19 is operated in correspondence with the manipulation in the manipulation unit 60 by a drive unit (not illustrated) provided in the adapter 18 (refer to FIG. 1). Surgical tools 29, 39, and 49 attached to the other slave manipulators 20, 30, and 40 may also have the same configuration as that of the above surgical tool 19.

Although not described in detail, the second manipulator 20, the third manipulator 30, and the fourth manipulator 40 illustrated in FIG. 1 have arms 21, 31, and 41 and adapters 28, 38, and 48, respectively, like the first manipulator 10, and the surgical tools 29, 39, and 49 are capable of being connected via the adapters 28, 38, and 48.

As illustrated in FIG. 1, manipulations for actuating the plurality of slave manipulators 10, 20, 30, and 40 and surgical tools 19, 29, 39, and 49 are input to the manipulation unit 60. The manipulation unit 60 has a master arm 61 configured so as to be held and manipulated by an operator with his/her hand. The amount of manipulation of the master arm 61 is detected in the input processing circuit 70.

The input processing circuit 70 analyzes manipulation signals from the manipulation unit 60, generates control signals (commands) for controlling the medical manipulator system 1 in accordance with analysis results of the manipulation signal, and inputs the generated control signal to the control unit 100.

The image processing circuit 80 performs various kinds of image processing for displaying image signals input from the control unit 100, and generates display image data in the display unit 90.

The display unit 90 composed of, for example, a liquid crystal display, and displays an image based on the image data generated in the image processing circuit 80 in accordance with image signals acquired via an observation instrument.

The control unit 100 illustrated in FIGS. 1 and 3 is, for example, a computer configured to have a CPU, a memory, and the like. The control unit 100 stores a predetermined program for controlling the plurality of slave manipulators 10, 20, 30, and 40, and controls the operation of the plurality of slave manipulators 10, 20, 30, and 40 and surgical tools 19, 29, 39, and 49 in accordance with the control signals (commands) from the input processing circuit 70.

The control unit 100 controls the plurality of slave manipulators 10, 20, 30, and 40 in accordance with one operation mode in the plurality of operation modes. In the following, the plurality of operation modes used in a case in which the control unit 100 operates the first manipulator 10 will be described focusing on a relationship between the first manipulator 10 and the second manipulator 20.

The plurality of operation modes for the control unit 100 to operate the first manipulator 10 include a first mode, a second mode, and a third mode.

The first mode is a mode in which the first manipulator 10 is operated only using the joints belonging to the normal joint 13A illustrated in FIG. 2. In the first mode, the control unit 100 (refer to FIG. 1) sets a path (first movement path) along which the first manipulator 10 can be moved only using the joints belonging to the normal joint 13A, on the basis of an operator's manipulation on the manipulation unit 60, and controls the first manipulator 10 such that the first manipulator 10 moves along the first movement path. In the present embodiment, a shortest path (a path in which that the amount of operation of the joint group 13 is minimized as a whole) to an arrival target position of the first manipulator 10 defined by a command generated on the basis of the manipulation in the manipulation unit 60 is adopted as a candidate for the first movement path, and the first movement path is determined as a movement path of the first manipulator 10 if any interference to be described below is not predicted.

The second mode is a mode in which the first manipulator 10 is operated using the joints belonging to the normal joint 13A illustrated in FIG. 2 and the joints belonging to the redundant joint 13B. In the second mode, the control unit 100 (refer to FIG. 1) sets a path (second movement path) along which the first manipulator 10 can be moved using both of the joints belonging to the normal joint 13A and the joints belonging to the redundant joint 13B, on the basis of the operator's manipulation on the manipulation unit 60, and controls the first manipulator 10 such that the first manipulator 10 moves along the second movement path. Selection of the second movement path in the second mode is subjected to limitation according to the position of the second manipulator 20. That is, the path set as the second movement path is set to a path that does not include the position of the second manipulator 20. Accordingly, the second movement path is set as a path that detours the position of the second manipulator 20. In addition, setting of the second movement path in the second mode is performed such that the first manipulator 10 can be moved without coming into contact with the second manipulator 20, also in consideration of the shape of the arm 21 of the second manipulator 20.

The third mode is a mode in which the first manipulator 10 is operated so as to cancel a contact state with an obstacle when the torque sensor 14 (refer to FIGS. 2 and 3) has detected the contact between the first manipulator 10 and the obstacle. In the present embodiment, in the third mode, the control unit 100 moves the first manipulator 10 in a direction in which the first manipulator 10 is separated from the obstacle.

Next, the configuration of the control unit 100 will be described. As illustrated in FIG. 3, the control unit 100 has a position recognition unit 101, a redundant control use determination unit 102, a return operation determination unit 105, a mode selection unit 107, and a drive signal generation unit 108. In the present embodiment, the program stored in the control unit 100 includes a program for making the control unit 100 function as the position recognition unit 101, the redundant control use determination unit 102, the return operation determination unit 105, the mode selection unit 107, and the drive signal generation unit 108.

The position recognition unit 101 recognizes the positions of the first manipulator 10 and the second manipulator 20. The positions of the first manipulator 10 and the second manipulator 20 are stored in the control unit 100 as coordinates in a coordinate system based on a predetermined origin in the medical manipulator system 1. In addition to the positions of the first manipulator 10 and the second manipulator 20, the position recognition unit 101 of the present embodiment may recognize the postures of the first manipulator 10 and the second manipulator 20.

The redundant control use determination unit 102 has an interference prediction unit 103 and an interference avoidance operation setting unit 104. The redundant control use determination unit 102 determines whether or not it is necessary to shift to the second mode. Moreover, the redundant control use determination unit 102 passes information for specifying the movement path (second movement path) of the first manipulator 10 in the second mode to the mode selection unit 107 when it has been determined to shift to the second mode.

The interference prediction unit 103 predicts whether or not the first manipulator 10 and the second manipulator 20 interfere with each other in a case in which the first manipulator 10 is moved along the above first movement path. The interference prediction unit 103 predicts that the first manipulator 10 and the second manipulator 20 may interfere with each other in a case in which the second manipulator 20 is located on the first movement path. The interference prediction unit 103 predicts that the first manipulator 10 and the second manipulator 20 do not interfere with each other in a case in which the second manipulator 20 is located out of the first movement path. For example, in the present embodiment, the interference prediction unit 103 recognizes a predetermined region including coordinates showing the position of the second manipulator 20 as a region in which the first manipulator 10 and the second manipulator 20 may interfere with each other, in consideration of the shape of the arm 21 of the second manipulator 20. Then, the interference prediction unit 103 predicts that the first manipulator 10 and the second manipulator 20 may interfere with each other in a case in which at least a portion of this region is located on the first movement path.

The interference avoidance operation setting unit 104 calculates a movement path between a start point and an end point of the first manipulator based on a command input from the manipulation unit 60 to the control unit 100 under the conditions of detouring the position of the second manipulator 20, and sets this movement path as the movement path (second movement path) of the first manipulator.

The return operation determination unit 105 detects whether or not the first manipulator 10 has come into contact with an obstacle with reference to the magnitude of a load in the torque sensor 14, and determines whether or not it is necessary to shift to the third mode. Additionally, the return operation determination unit 105 has a return operation setting unit 106 that sets the amount of operation of the first manipulator 10 in the third mode.

The mode selection unit 107 selects one mode from the plurality of operation modes (the first mode, the second mode, and the third mode) for operating the control unit 100, and the control unit 100 is operated in accordance with the selected mode. The mode selection unit 107 of the present embodiment selects the first mode in a case in which the interference prediction unit 103 has predicted if the first manipulator 10 do not interfere with the second manipulator 20. Additionally, in a case in which the interference prediction unit 103 has predicted if the first manipulator 10 interferes with the second manipulator 20, the redundant control use determination unit 102 determines that the shift to the second mode is required. Thus, the mode selection unit 107 selects the second mode. Moreover, in a case in which the torque sensor 14 has detected the contact between the first manipulator 10 and the obstacle while the control unit 100 is operated in the first mode or the second mode, the return operation determination unit 105 determines that shift of the third mode from the first mode or the second mode is required. Thus, the mode selection unit 107 selects the third mode.

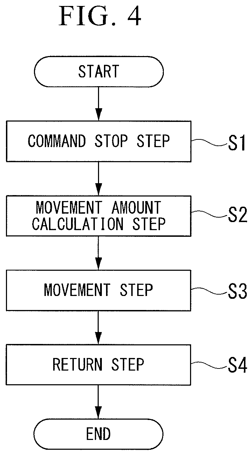

In the present embodiment, in a case in which the mode selection unit 107 selects the third mode, the control unit 100 is operated in a control procedure shown in respective following steps. FIG. 4 is a flowchart illustrating the control procedure in the control unit 100 of the medical manipulator system 1 of the present embodiment.

The control unit 100 illustrated in FIG. 3 first discontinues the control of the first manipulator 10 by the first mode and the second mode (Step S1, a command stop step, and refer to FIG. 4). In this case, the mode selection unit 107 saves a command output, based on a manipulation on the manipulation unit 60, from the input processing circuit 70 to the control unit 100 in a buffer (primary memory) (not illustrated) of the control unit 100. Accordingly, the control unit 100 stops the control of the first manipulator 10 based on the command. The command saved in the buffer of the control unit 100 is read at the time of return to an operation mode before the shift (first mode or second mode) from the third mode, and is subsequently used as a command to the control unit 100 for operating the first manipulator 10.

After Step S1, the return operation setting unit 106 of the control unit 100 calculates a movement amount of the first manipulator 10 for canceling a contact state with the obstacle (Step S2, a movement amount calculation step). In this case, the control unit 100, for example, calculates the movement amount of the first manipulator 10 such that the first manipulator 10 moves toward a side opposite to a direction in which the first manipulator 10 has come into contact with the obstacle.

After Step S2, the control unit 100 outputs a drive signal from the drive signal generation unit 108 to the first manipulator 10 on the basis of the movement amount calculated in Step S2, thereby moving the first manipulator 10 by the above movement amount (Step S3, a movement step).

After Step S3, the control unit 100 returns to a mode before the shift to the third mode, out of the first mode and the second mode (Step S4, a return step). In this case, the control unit 100 reads the command saved in the above buffer, thereby setting a movement target position of the first manipulator 10, and controls the first manipulator 10 so as to move the first manipulator 10 to the movement target position.

The control unit 100 controls the operations of the slave manipulator 10 and the like, which are manipulation targets of the manipulation unit 60, on the basis of the command. In this case, the control unit 100 is able to output drive signals to the corresponding slave manipulator 10 and the like, and operate the slave manipulator 10 and the like that are manipulation targets while the driving amounts and loads of the slave manipulator 10 and the like that are the manipulation targets, in accordance with detection signals input from the torque sensor 14 and the encoder 16 in accordance with the operations of the corresponding slave manipulator 10 and the like.

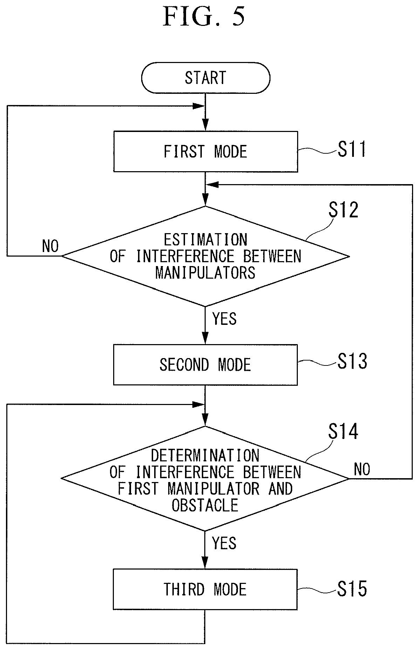

The control of the first manipulator 10 by the control unit 100 will be described in detail with reference to FIG. 1 to FIGS. 3 and 5. FIG. 5 is a flowchart illustrating the flow of a manipulation using the medical manipulator system 1 of the present embodiment.

The control unit 100 illustrated in FIG. 3 controls the first manipulator 10 in accordance with an operation mode selected by the mode selection unit 107. An initial operation mode in the mode selection unit 107 is the first mode (Step S11, refer to FIG. 5). If the operation of the control unit 100 is started in the first mode in Step S11, the process proceeds to Step S12.

Step S12 is a step in which the mode selection unit 107 refers to a prediction result in the interference prediction unit 103 and branches processing in accordance with the prediction result. In Step S12, the processing proceeds to Step S13 if it is determined that the second manipulator 20 (refer to FIG. 2) is located on the path (first movement path) set in the first mode (that is, it is predicted that the first manipulator 10 and the second manipulator 20 may interfere with each other). In Step S12, if it is determined that the second manipulator 20 is located out of the path (first movement path) set in the first mode, the processing returns to Step S11 in which the first mode is continued.

Step S13 is a step in which the mode selection unit 107 selects the second mode to operate the control unit 100 in the second mode. In the second mode, as the first manipulator 10 moves along the second movement path about which the position of the second manipulator 20 is taken into consideration, the first manipulator 10 is movable without interfering with the second manipulator 20. If the operation of the control unit 100 is started in the second mode in Step S13, the processing proceeds to Step S14.

Step S14 is a step in which the torque sensor 14 illustrated in FIG. 3 refers to the magnitude of a load detected by the return operation determination unit 105, and branches processing in accordance with magnitude of the load. In Step S14, in a case in which the magnitude of the load detected by the torque sensor 14 exceeds a predetermined threshold value, it is determined that the first manipulator 10 has come into contact with an obstacle not recognized by the medical manipulator system 1, and the processing proceeds to Step S15. In Step S14, in a case in which the magnitude of the load detected by the torque sensor 14 exceeds the predetermined threshold value, it is determined that the first manipulator 10 does not come into contact with the obstacle, and the processing returns to Step S12.

Step S15 is a step in which the mode selection unit 107 selects the third mode to operate the control unit 100 in the third mode. In the third mode, the control unit 100 moves the first manipulator 10 in a direction in which a contact state between the first manipulator 10 and the obstacle is cancelled. In Step S15, the first manipulator 10 is moved on the basis of the amount of operation set by the return operation setting unit 106, and the processing returns to Step S14.

By virtue of the repetition of Step S14 and Step S15, even in a case in which the amount of operation of the first manipulator 10 is insufficient and a contact state with the obstacle is not cancelled, the first manipulator 10 is moved until a contact state with the obstacle is cancelled, and the processing returns to Step S12 after a contact state with the obstacle is cancelled. In addition, in the repetitive operation of Step S14 and Step S15, the first manipulator 10 may be moved by a predetermined minute amount in the direction in which a contact state between the first manipulator 10 and the obstacle is cancelled, not limited to the amount of operation set by the return operation setting unit 106.

By virtue of the respective steps from the above Step S11 to the above Step S15, the control unit 100 shifts the first mode, the second mode, and the third mode, and controls the first manipulator 10 in accordance with any one operation mode among the respective operation modes.

In addition, in a case in which the contact between the first manipulator 10 and the obstacle has been detected during movement in the first mode, the first mode may be directly shifted to the third mode (return mode). In the case, if a contact state between the first manipulator 10 and the obstacle is cancelled, the processing proceeds to the first mode.

Although details are omitted, the control of the first manipulator 10 by the control unit 100 illustrated in FIG. 1 is performed in consideration of the positions of the third manipulator 30 and the fourth manipulator 40 in addition to the position of the second manipulator 20. Additionally, in a case in which the second manipulator 20 is a control object, the second manipulator 20 is controlled by the control unit 100 in consideration of the positions of the first manipulator 10, the third manipulator 30, and the fourth manipulator 40. The same applies to a case in which the third manipulator 30 and the fourth manipulator 40 are control objects of the control unit 100. That is, in the present embodiment, the four slave manipulators are controlled by the control unit 100 so as to avoid any interference therebetween on the basis of their mutual positions. Moreover, when the slave manipulators have come into contact with an obstacle, the slave manipulators are controlled by the control unit 100 so as to automatically cancel a contact state.

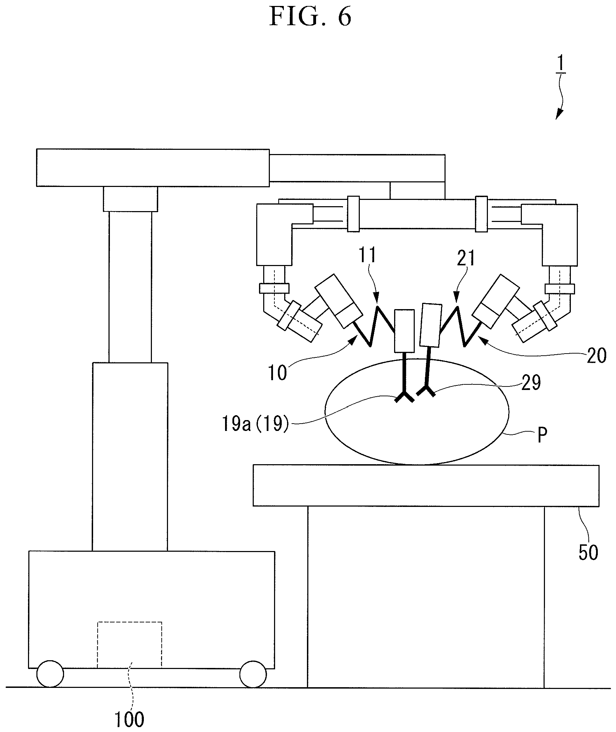

The working of the medical manipulator system 1 of the present embodiment will be described. FIGS. 6 to 8 are views for explaining the working of the medical manipulator system 1 of the present embodiment.

The medical manipulator system 1 of the present embodiment is used in a state in which surgical tools 19 and 29 are attached to the first manipulator 10 and the second manipulator 20. In addition, if necessary, an endoscope may be combined with the medical manipulator system 1 of the present embodiment. In this case, the operation of the endoscope may be controlled by the control unit 100. The endoscope combined with the medical manipulator system 1 of the present embodiment images a site inside the body used as a target where a treatment is performed using surgical tools (the first surgical tool 19, the second surgical tool 29) attached to the first manipulator 10 and the second manipulator 20, and displays an endoscopic image on the display unit 90 (refer to FIG. 1).

When using the medical manipulator system 1 illustrated in FIGS. 1 and 6, a treatment is performed on a treatment target site while moving the respective surgical tools 19 and 29 using the first manipulator 10 and the second manipulator 20. Here, in order to move the first surgical tool 19 attached to the first manipulator 10, for example, an operator performs a manipulation for moving the first surgical tool 19 on the manipulation unit 60. Although the operator who manipulates the manipulation unit 60 views the endoscopic image displayed on the display unit 90 and grasps the position and the posture of an end effector 19a at a distal end of the first surgical tool 19, the operator may not grasp the position of the arm 11 of the first manipulator 10. In this case, the manipulation on the manipulation unit 60 is performed without taking into consideration a possibility that a portion of the arm 11 of the first manipulator 10 may interfere with the second manipulator 20.

In the present embodiment, if the control unit 100 receives a command generated on the basis of the manipulation in the manipulation unit 60, the control unit 100 calculates the first movement path for moving the first manipulator 10 only using the normal joint 13A (refer to FIG. 2) on the basis of the command. Moreover, as illustrated in the above Steps S11 to S15, the control unit 100 predicts the presence/absence of any potential interference between the first manipulator 10 and the second manipulator 20, and controls the first manipulator 10 in the first mode or the second mode.

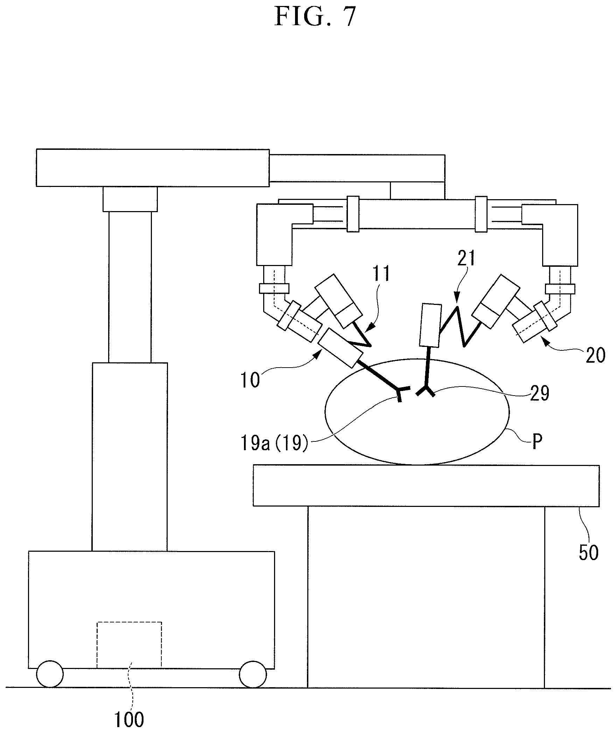

In the first mode, the first manipulator 10 is moved so as to take a position and a posture according to the command only using the normal joint 13A illustrated in FIG. 2. As a result, the end effector 19a of the first surgical tool 19 takes a position and a posture (for example, refer to FIG. 7) corresponding to the manipulation on the manipulation unit 60. The position and the posture after the movement of the end effector 19a of the first surgical tool 19 in this case may be obtained, for example, by performing a pivot movement with an insertion point of the first surgical tool 19 as a remote center or by performing a parallel movement or a rotational movement.

In the second mode, the first manipulator 10 is moved so as to detour the position of the second manipulator 20, also using the redundant joint 13B in addition to the normal joint 13A illustrated in FIG. 2. As a result, the end effector 19a of the first surgical tool 19 takes a position and a posture corresponding to the manipulation on the manipulation unit 60 (refer to FIG. 1). In the second mode, if the manipulation on the manipulation unit 60 is the same as the manipulation in the above first mode, the posture of the arm 11 of the first manipulator 10 after the movement is different from that in the first mode. However, the position and the posture of the end effector 19a of the first surgical tool 19 after the movement can be made to be substantially the same as that in the first mode.

In this way, in the present embodiment, even in the first mode and the second mode, any interference between the first manipulator 10 and the second manipulator 20 is not caused, and the first surgical tool 19 takes a position and a posture corresponding to the manipulation on the manipulation unit 60.

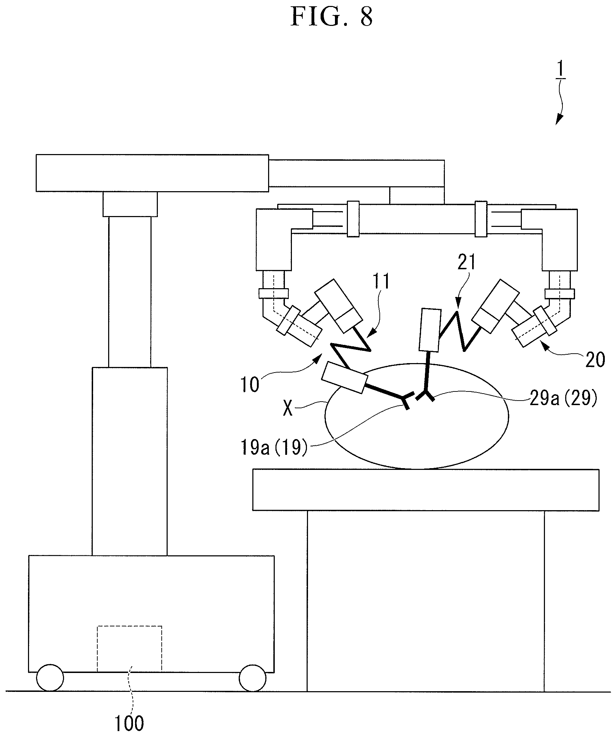

Meanwhile, in the first mode and the second mode, the first manipulator 10 is moved. Thus, a possibility that the first manipulator 10 comes into contact with an obstacle X (refer to FIG. 8) not recognized by the medical manipulator system 1 of the present embodiment is considered. For example, as illustrated in FIG. 8, a possibility that the first manipulator 10 comes into contact with an abdominal wall (obstacle X) of a patient P is considered. The position of the patient P in a space where the medical manipulator system 1 of the present embodiment is installed, the positions of medical instruments (not illustrated) other than the medical manipulator system 1, the position of an assistant who assists in the manipulation of the medical manipulator system 1 of the present embodiment, or the like may vary during surgical operation. Thus, it is difficult to grasp all these positions in real time.

In the medical manipulator system 1 of the present embodiment, in a case in which the first manipulator 10 has come into contact with objects other than the object that is recognized in advance by the medical manipulator system 1, the torque sensor 14 (refer to FIG. 3) that is a contact detection unit detects the presence of the object, and the control unit 100 recognizes the object as an obstacle. Then, when the first manipulator 10 has come into contact with the obstacle, the control unit 100 shifts from the first mode or the second mode to the third mode in accordance with a selection performed by the mode selection unit 107, and operates the first manipulator 10 so as to cancel a contact state with the obstacle.

In the third mode, the control unit 100 moves the first manipulator 10 such that the first manipulator 10 is separated from the obstacle (refer to Steps S1 to S4 illustrated in FIG. 4). In the medical manipulator system 1 of the present embodiment, if the torque sensor 14 stops detecting the contact between the first manipulator and the obstacle, it is possible to automatically return from the third mode to the mode before the shift (first mode or second mode).

For example, in a case in which the processing has returned from the third mode to the first mode, control that moves the first manipulator 10 in the shortest path on the basis of the manipulation on the manipulation unit 60 illustrated in FIG. 1 is performed only using the normal joint 13A (refer to FIG. 2).

For example, in a case in which the processing has returned from the third mode to the second mode, the control of moving the first manipulator 10 in a path about which the position of the second manipulator 20 is taken into consideration, on the basis of the manipulation on the manipulation unit 60 illustrated in FIG. 1, using both the normal joint 13A and the redundant joint 13B (refer to FIG. 2), is performed.

As described above, according to the medical manipulator system 1 of the present embodiment, even if any of the first mode and the second mode is selected, the first surgical tool 19 can take a desired position and a desired posture without the first manipulator 10 interfering with the second manipulator 20. Moreover, according to the medical manipulator system 1 of the present embodiment, even if the first manipulator 10 has come into contact with an obstacle during the movement of the first manipulator 10, the first surgical tool 19 can be made to take a desired position and a desired posture by automatically canceling a contact state between the first manipulator 10 and the obstacle. As a result, according to the medical manipulator system 1 of the present embodiment, even if an obstacle is on the movement path for avoiding any interference between the arm 11 of the first manipulator 10 and the arm 21 of the second manipulator 20, the interference can be rapidly avoided. In this way, since the interference is automatically avoided, an operator who manipulates the manipulation unit 60 can concentrate on manipulating the end effector 19a displayed on the display unit 90 to perform a suitable treatment on a treatment target site, without taking into consideration the presence of arms 11 and 21 or the movement and interference thereof.

### (Modification Examples)

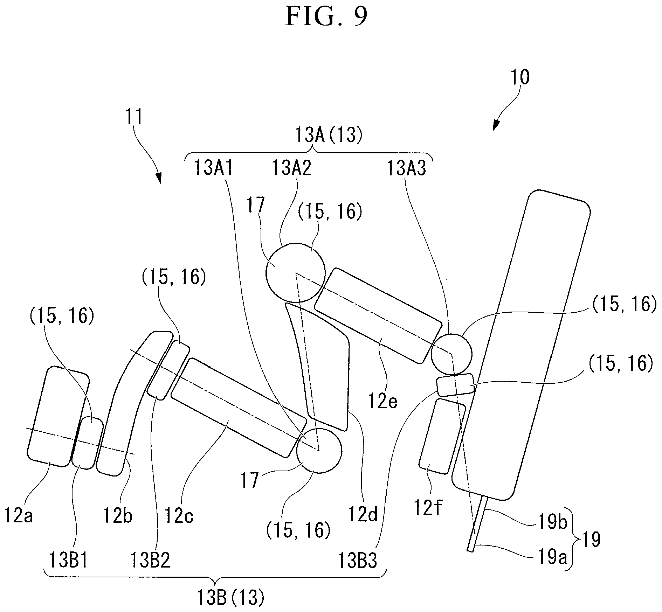

A modification example of the above first embodiment will be described. FIG. 9 is a schematic view illustrating the first manipulator in the present modification example.

In the present modification example, as the contact detection unit, a contact sensor 17 is provided as illustrated in FIG. 9 instead of the torque sensor 14 (refer to FIG. 2) disclosed in the first embodiment.

The contact sensor 17 is disposed on an outer surface of the arm 11 of the first manipulator 10. Additionally, the contact sensor 17 is connected to the control unit 100. As an object that is not recognized at the time of starting to use the medical manipulator system 1 comes into contact with the contact sensor 17, this object is recognized as an obstacle by the contact sensor 17 and the control unit 100.

In the present modification example, in a case in which an obstacle has come into contact with the contact sensor 17, the first manipulator 10 can be operated so as to cancel a contact state between the obstacle and the first manipulator 10, as in the above first embodiment.

In addition, both the torque sensor 14 and the contact sensor 17 may be provided as the contact detection unit of the present modification example. In this case, the contact between the first manipulator 10 and the obstacle can be detected by the torque sensor 14 even in a region where the contact sensor 17 is not attached in the arm 11 of the first manipulator 10, and the contact with the obstacle can be detected without applying the torque generated by the actuator 15 of the first manipulator 10, in a region where the contact sensor 17 is attached.

### (Second Embodiment)

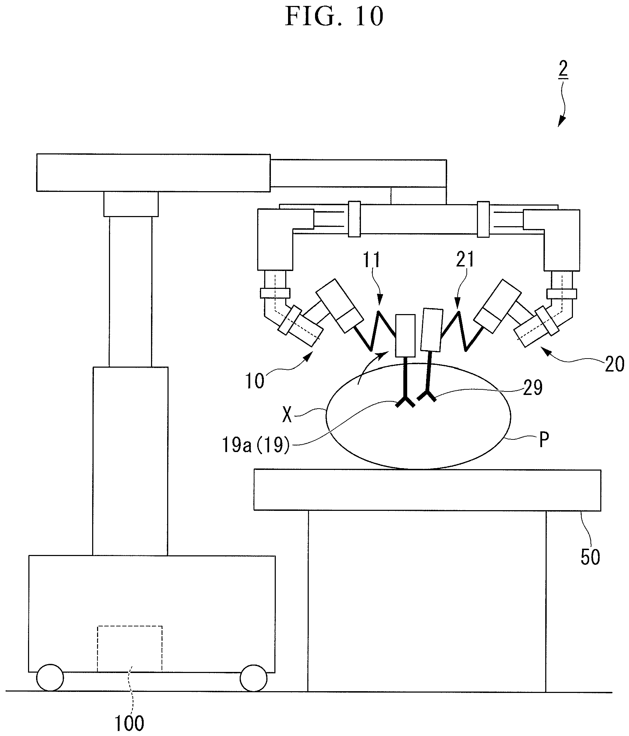

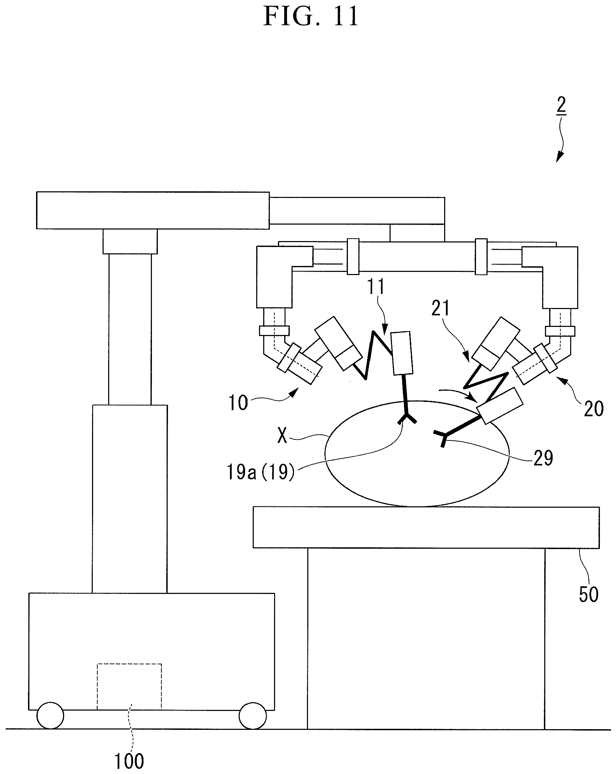

A second embodiment of the invention will be described. FIG. 10 is a schematic view of a medical manipulator system of the present embodiment. FIG. 11 is a view for explaining the working of the medical manipulator system of the present embodiment.

The medical manipulator system 2 of the present embodiment is different from that of the above first embodiment in terms of the operation of the control unit 100 in the third mode.

The second manipulator 20 moves the medical manipulator system 2 of the present embodiment in addition to the first manipulator 10 in the third mode. That is, the second manipulator 20 can be operated independently from the first manipulator 10 or in cooperation with the first manipulator 10 by the control performed by the control unit 100.

For example, as illustrated in FIG. 10, when the first manipulator 10 is moving in a direction away from the obstacle X in the third mode, as illustrated in FIG. 11, the second manipulator 20 is moved in a direction in which the second manipulator 20 is separated from the first manipulator 10. Accordingly, in the present embodiment, the first manipulator 10 and the second manipulator 20 are at positions separated from each other when a contact state between the first manipulator 10 and an obstacle has been cancelled in the third mode. Thus, the probability that the first manipulator 10 interferes with the second manipulator 20 due to the movement of the first manipulator 10 after that can be lowered.

Additionally, the control unit 100 may analyze a command based on a manipulation that the operator has performed on the manipulation unit 60, and may retract the second manipulator 20 out of the movement path of the first manipulator 10 after the end of the third mode. In this case, after the second mode shifts to the third mode, the processing proceeds to the first mode without returning to the second mode.

In the present embodiment, as the second manipulator 20 cooperates with the first manipulator 10, the alternative of the movement path of the first manipulator 10 is expanded. Thus, a possibility that another interference is caused due to the movement of the arm 11 for avoiding any interference can be kept low.

### (Third Embodiment)

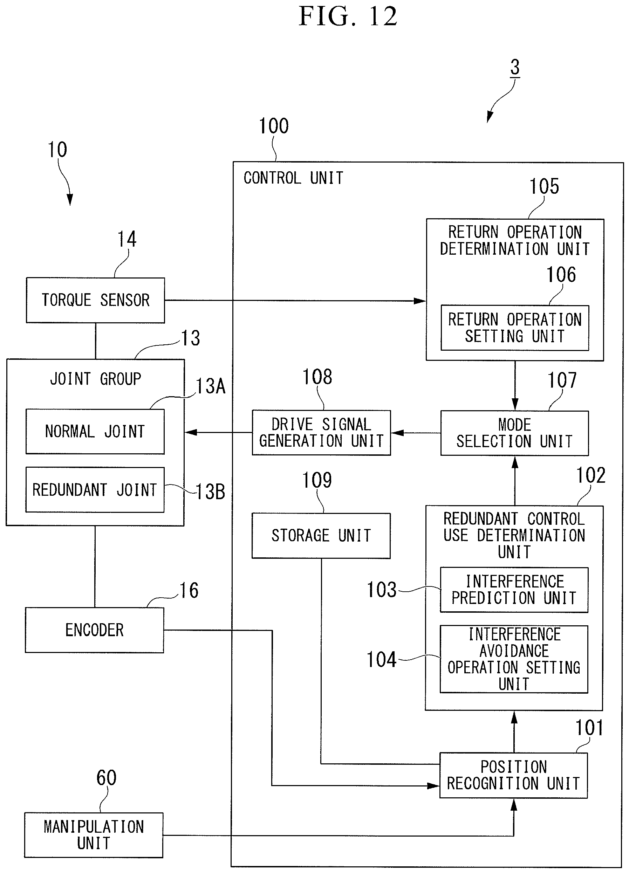

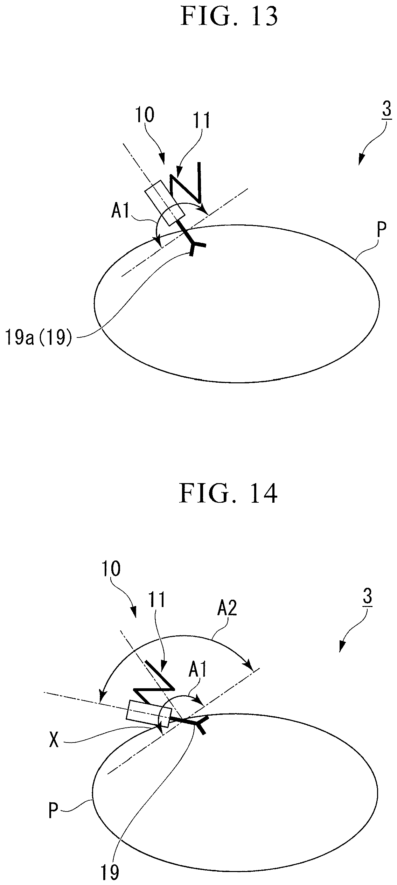

A third embodiment of the invention will be described. FIG. 12 is a block diagram illustrating main units of a medical manipulator system of the present embodiment. FIGS. 13 and 14 are views for explaining the working of the medical manipulator system of the present embodiment.

In the medical manipulator system 3 of the present embodiment, as illustrated in FIG. 12, the control unit 100 is different from the above first embodiment in terms of configuration in that, when the first manipulator 10 comes into contact with an object that is not recognized at the time of starting to use the medical manipulator system 3, the control unit has a storage unit 109 that recognizes as an obstacle of the object to store the position thereof.

Additionally, the control unit 100 of the medical manipulator system 3 of the present embodiment uses the coordinates of the obstacle stored in the storage unit 109 as an entry prohibition position of the first manipulator 10.

For example, in a case in which the coordinates used as the entry prohibition position of the first manipulator 10 are not stored in the storage unit 109, the first manipulator 10 is movable throughout a maximum movable range (for example, illustrated by reference sign A1 in FIG. 13 as an example) of the surgical tool 19 by the arm 11.

Here, if the first manipulator 10 moves and comes into contact with the obstacle X (a body wall of the patient P in the present embodiment) as illustrated in FIG. 14, the coordinates used as the entry prohibition position of the first manipulator 10 are stored in the storage unit 109, and a movable range of the first manipulator 10 is limited to a range A2 up to a position where the first manipulator 10 has come into contact with the obstacle X in the maximum movable range A1 of the surgical tool 19 by the arm 11. Accordingly, the entry prohibition position of the first manipulator 10 is set, and the movable range of the first manipulator 10 continues being limited after the setting of the entry prohibition position.

In a state in which the entry prohibition position of the first manipulator 10 is set, the control unit 100 (refer to FIG. 12) of the present embodiment sets the first movement path so as to detour the entry prohibition position as a control according to the first mode. In a case in which the entry prohibition position cannot be detoured (in a case in which the setting of the first movement path cannot be performed) in using only the normal joint 13A (refer to FIG. 2), a shift to the second mode is performed. Additionally, in a state in which the entry prohibition position of the first manipulator 10 is set, the control unit 100 of the present embodiment sets the second movement path so as to detour the entry prohibition position and the position of the second manipulator 20 as a control according to the second mode.

The storage of the coordinates of the entry prohibition position in the storage unit 109 is held until the end of use of the medical manipulator system 3. Additionally, if necessary, by deleting the coordinates of an arbitrary entry prohibition position, the first manipulator 10 may be allowed to enter a position, which has been the entry prohibition position, again. For example, in a case in which the position of an assistant who is near the first manipulator 10 in using the medical manipulator system 3 is stored as the entry prohibition position, limitation on the movable range of the first manipulator 10 can be eliminated by deleting the coordinates from the storage unit after the assistant has moved from the position thereof.

The medical manipulator system 3 of the present embodiment exhibits the same effects as those of the above first embodiment. Additionally, in the present embodiment, an obstacle recognized after the start of use of the medical manipulator system 3 does not come into contact after that. Thus, useless operation of the first manipulator 10 is reduced.

In addition, in the present embodiment, in a case in which a big problem is not posed even if an obstacle comes into contact, the entry prohibition position may be allowed to be included in coordinates through which the first manipulator 10 passes when selecting a movement path of the first manipulator 10. In this case, the movement path including the entry prohibition position is lower in priority than a movement path not including the entry prohibition position. Accordingly, the control unit 100 allows the first manipulator 10 to pass through the entry prohibition position only in a case in which the movement path not including the entry prohibition position cannot be set.

### (Fourth Embodiment)

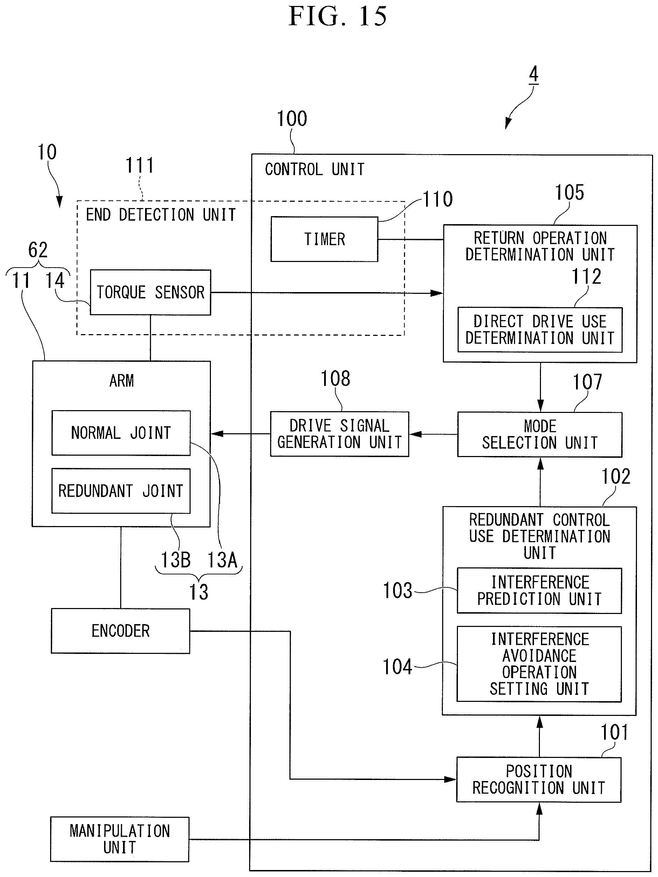

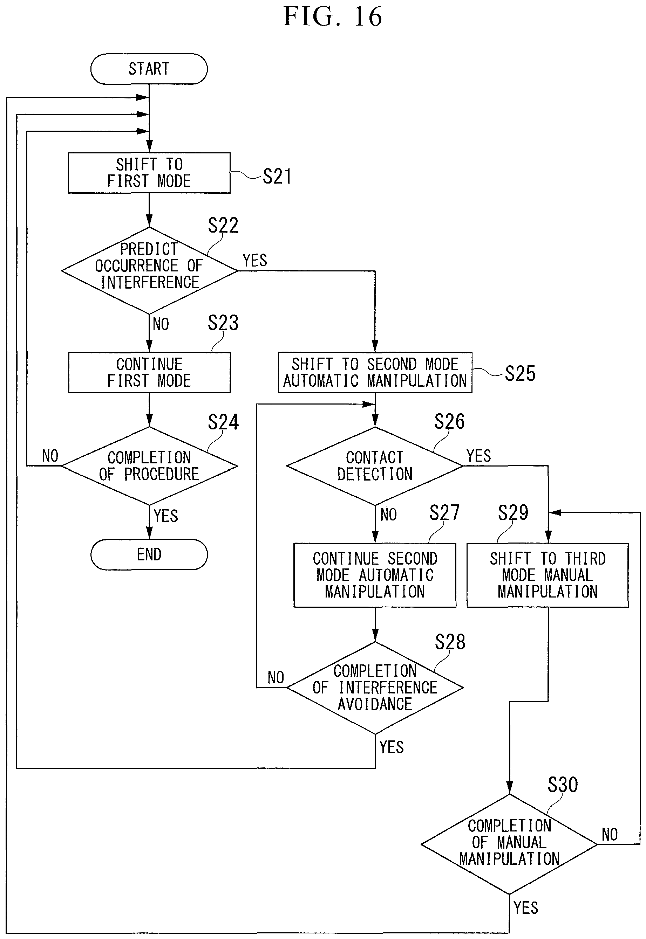

A fourth embodiment of the invention will be described. FIG. 15 is a block diagram illustrating main units of a medical manipulator system of the present embodiment. FIG. 16 is a flowchart illustrating the flow of a manipulation using the medical manipulator system of the present embodiment.

The medical manipulator system 4 (refer to FIG. 15) of the present embodiment is different from the medical manipulator system 1 disclosed in the above first embodiment in terms of the contents of the third mode.

The third mode in the present embodiment is a direct drive mode in which the first manipulator 10 (refer to FIG. 2) is not automatically operated but an assistant is allowed to operate the first manipulator 10 with his/her manual manipulation near the first manipulator 10.

In the present embodiment, as illustrated in FIG. 15, the arm 11 and the torque sensor 14 of the first manipulator 10 serve as a second manipulation unit 62 capable of manipulating the first manipulator 10 by a method different from the manipulation unit 60. In the present embodiment, the return operation determination unit 105 of the control unit 100 has a direct drive use determination unit 112 that determines whether or not to perform a manipulation using the second manipulation unit 62, instead of the return operation setting unit 106.

As an example, the control unit 100 of the present embodiment in a state in which the third mode is selected first saves a command in the buffer as in the first embodiment, thereby stopping the control of the first manipulator 10 based on the command (command stop step).

Moreover, the direct drive use determination unit 112 of the control unit 100 releases a brake for the joint group 13 of the first manipulator 10, and permits the first manipulator 10 to operate to follow up an input in a case in which there is the input to the torque sensor 14 of the arm 11.

That is, in the third mode of the present embodiment, it is a manipulation for the torque sensor 14 in which an assistant pushes and pulls or rotates the arm 11 of the first manipulator 10. The manipulation for the torque sensor 14 is a command for moving the arm 11 with the actuator 15 in a direction in which the assistant pushes and pulls the arm 11. In this way, in the present embodiment, the control unit 100 permits the manipulation of the first manipulator by the second manipulation unit 62 through the release of the brake of the joint group 13 and the follow-up operation control of the first manipulator 10 (permission step).

If necessary, the control unit 100 of the present embodiment may constrain an insertion point (a remote center RC, refer to FIG. 2) of the first surgical tool 19, may constrain some joints of the joint group 13 that connect the link group 12 of the arm 11, or may make all the joints freely movable.

Additionally, as illustrated in FIG. 15, the control unit 100 is provided with a timer 110 that counts the time for which there is no input to the torque sensor 14. After lapse of a given time since an input to the torque sensor 14 has disappeared, the control unit 100 determines that the manipulation for the first manipulator 10 by the assistant has ended. That is, the torque sensor 14 and a timer 110 constitutes an end detection unit 111 that detects the end of the manipulation performed by the second manipulation unit 62.

After the end detection unit 111 detects the end of the manipulation performed by the second manipulation unit 62, the control unit 100 ends the third mode to return to the first mode or the second mode (return step).

The operation of the medical manipulator system 4 of the present embodiment will be described in detail along a flowchart illustrated in FIG. 16.

In the present embodiment, after the start of the medical manipulator system 4, first, a shift to the first mode is performed and the control unit 100 operates in the first mode (Step S21, refer to FIG. 16). Subsequently, the redundant control use determination unit 102 predicts occurrence of interference between the first manipulator 10 and the second manipulator 20 (Step S22). In a case in which it is predicted that there is not interference in Step S22, the first mode is continued (Step S23) and it is determined whether or not a procedure is completed (Step S24). In Step S24, if a procedure is being performed, the processing returns to Step S21 in which the first mode is further continued, and if the procedure is completed, a series of steps end.

In a case in which it is predicted that interference may occur in the above Step S22, the first mode shifts to the second mode (Step S25). After a shift to the second mode has been performed by Step S25, the first manipulator 10 is controlled by the control unit 100 so as to avoid any interference with the second manipulator 20.

After Step S25, whether or not the first manipulator 10 has come into contact with an obstacle is detected (Step S26). In Step S26, in a case in which the contact between the first manipulator 10 and the obstacle is not detected, the processing proceeds to Step S27 in which the second mode is continued, and it is determined whether or not the first manipulator 10 has reached the arrival target position while avoiding any interference with the second manipulator 20 and interference avoidance has been completed (Step S28). If the interference avoidance is not completed in Step S28, the processing returns to Step S26, and if the interference avoidance is completed in Step S28, the processing returns to Step S21.

In the above Step S26, in a case in which the contact between the first manipulator 10 and an obstacle is detected, the processing proceeds to Step S29 and the second mode shifts to the third mode. The third mode in the present embodiment is a mode in which the arm 10 can be moved more directly than in a case in which the manipulation unit 60 is used in that an assistant directly moves the arm 11 of the first manipulator 10 to separate the arm from an obstacle with his/her manual manipulation.

While the assistant is performing the manual manipulation of the arm 11 in Step S29, the end detection unit 111 continues (Step S30) determining whether or not the manual manipulation has ended. In the present embodiment, the end detection unit 111 determines that the manual manipulation is completed with the movement manipulation for the arm 11 not being performed for a given time or more. If the end detection unit 111 determines that the manual manipulation has been completed, the processing returns to Step S21 in which operation is started in the first mode.

According to the medical manipulator system 4 of the present embodiment, even in a case in which the movement of the arm 11 for canceling a contact state between an obstacle and the first manipulator 10 is complicated and cannot be automatically cancelled, the arm 11 can be moved at the assistant's discretion. Thus, it is easy to cancel a contact state between the obstacle and the first manipulator 10.

### (Modification Example 1)

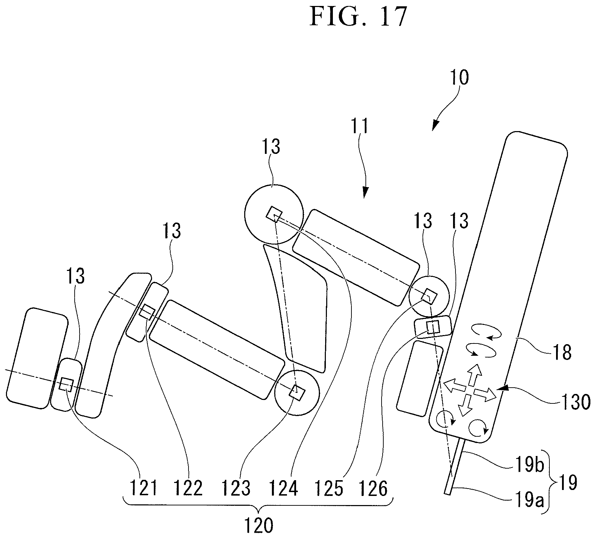

Modification Example 1 of the above fourth embodiment will be described. FIG. 17 is a schematic view illustrating the first manipulator in the present modification example.

In the present modification example, as illustrated in FIG. 17, the first manipulator 10 has an indicator 120 showing how the respective joints are controlled.

For example, the first manipulator 10 has lamps 121 to 126 indicating three states including "constrained (not moved)", "operated according to a manipulation performed by the first manipulation unit", and "automatically controlled in order to avoid any interference with other manipulators" in different colors, respectively, as indicators at the respective joints of the joint group 13.

The lamps 121 to 126 disposed at the respective joints of the joint group 13 make an assistant easily ascertain current states of the joints in the colors of he lamps 121 to 126. Accordingly, in the present modification example, the assistant can ascertain which joints can move in advance, and the assistant can easily evacuate such that the arm 11 and the assistant do not come into contact with each other.

In addition, the indicator 120 may be provided at the other manipulators 20, 30, and 40 (refer to FIG. 1).

### (Modification Example 2)

Modification Example 2 of the above fourth embodiment will be described.

In the present modification example, as illustrated in FIG. 17, the first manipulator 10 has a navigator 130 indicating in which direction it is preferable for an assistant to move the arm 11 in the third mode. Additionally, in the present modification example, a control unit (not illustrated) controls the navigator 130 on the basis of a positional relationship between the manipulator 10, the second manipulator, and an obstacle.

In the present modification example, the control unit outputs, to the navigator 130, a signal a movement recommendation direction of the first manipulator 10 so as to move the first manipulator 10 to a substantially intermediate position between an obstacle and the second manipulator on the basis of the position of the obstacle and the position of the second manipulator.

The navigator 130 has, for example, an arrow-shaped lamp, a liquid crystal display monitor, or the like and displays the movement recommendation direction of the first manipulator 10, for example, by an arrow.

In the present modification example, the navigator 130 helps to move the first manipulator 10 to a position farthest from the obstacle and the second manipulator between the obstacle and the second manipulator. Thus, the assistant only has to move the first manipulator 10 as being displayed on the navigator 130, so that the manipulation can be simplified.

Although the embodiments of the invention have been described above in detail with reference to the drawings, specific configuration is not limited to these embodiments, and design changes are also included without departing from the scope of the invention.

Additionally, the constituent elements illustrated in the above-described respective embodiments and respective modification examples can be suitably configured in combination.

### Industrial Applicability

The invention is applicable to a manipulator system.

### Reference Signs List

- 1, 2, 3, 4:: MEDICAL MANIPULATOR SYSTEM
- 10:: FIRST MANIPULATOR (SLAVE MANIPULATOR)
- 11:: ARM
- 12:: LINK GROUP
- 13:: JOINT GROUP
- 13A:: NORMAL JOINT
- 13B:: REDUNDANT JOINT
- 14:: TORQUE SENSOR (CONTACT DETECTION UNIT)
- 15:: ACTUATOR
- 16:: ENCODER
- 17:: CONTACT SENSOR (CONTACT DETECTION UNIT)
- 18:: ADAPTER
- 19:: SURGICAL TOOL (FIRST SURGICAL TOOL)
- 19a:: END EFFECTOR
- 20:: SECOND MANIPULATOR (SLAVE MANIPULATOR)
- 21:: ARM
- 28:: ADAPTER
- 29:: SURGICAL TOOL (SECOND SURGICAL TOOL)
- 30:: THIRD MANIPULATOR (SLAVE MANIPULATOR)
- 31:: ARM
- 38:: ADAPTER
- 39:: SURGICAL TOOL
- 40:: FOURTH MANIPULATOR (SLAVE MANIPULATOR)
- 41:: ARM
- 48:: ADAPTER
- 49:: SURGICAL TOOL
- 50:: SURGICAL BED
- 60:: MANIPULATION UNIT
- 61:: MASTER ARM
- 62:: SECOND MANIPULATION UNIT
- 70:: INPUT PROCESSING CIRCUIT
- 80:: IMAGE PROCESSING CIRCUIT
- 90:: DISPLAY UNIT
- 100:: CONTROL UNIT
- 101:: POSITION RECOGNITION UNIT
- 102:: REDUNDANT CONTROL USE DETERMINATION UNIT
- 103:: INTERFERENCE PREDICTION UNIT
- 104:: INTERFERENCE AVOIDANCE OPERATION SETTING UNIT
- 105:: RETURN OPERATION DETERMINATION UNIT
- 106:: RETURN OPERATION SETTING UNIT
- 107:: MODE SELECTION UNIT
- 108:: DRIVE SIGNAL GENERATION UNIT
- 109:: STORAGE UNIT
- 110:: TIMER
- 111:: END DETECTION UNIT
- 112:: DIRECT DRIVE USE DETERMINATION UNIT
- 120:: INDICATOR
- 121 TO 126:: LAMP
- 130:: NAVIGATOR

## Claims

1. A medical manipulator system comprising:
a first manipulator having a joint group;
a first manipulation unit that issues a command for manipulating the first manipulator;
a control unit that receives the command and controls the first manipulator according to one operation mode among a plurality of operation modes;
a contact detection unit that detects that the first manipulator and an obstacle have come into contact with each other; and
a second manipulator capable of operating independently from or in cooperation with the first manipulator;
wherein the joint group includes
one or more joints belonging to a first group, and
one or more joints belonging to a second group,
wherein the plurality of operation modes includes
a first mode in which the first manipulator is controlled such that the first manipulator moves along a first movement path on which the first manipulator is operated only using the joints belonging to the first group,
a second mode in which the first manipulator is controlled such that the first manipulator moves along the second movement path on which the first manipulator is moved using the joints belonging to the first group and the joints belonging to the second group, and
a third mode in which, when the contact detection unit detects a contact between the first manipulator and the obstacle, the first manipulator is operated so as to cancel a contact state between the first manipulator and the obstacle,
wherein the control unit includes
a position recognition unit that recognizes positions of the first manipulator and the second manipulator,
an interference prediction unit that predicts whether or not the first manipulator interferes with the second manipulator in a case in which the first manipulator is moved along the first movement path, and
a mode selection unit that selects any one of the first mode and the second mode such that first mode is selected in a case in which the interference prediction unit predicts that the first manipulator does not interfere with the second manipulator, and second mode is selected in a case in which the interference prediction unit predicts that the first manipulator interferes with the second manipulator, and selects one mode from plurality of operation modes so as to shift from the first mode or the second mode to the third mode in a case in which the contact detection unit detects the contact between the first manipulator and the obstacle while the control unit is operating in the first mode or the second mode, and
wherein the control unit sets a path for detouring the position of the second manipulator as the second movement path in the second mode.

2. The medical manipulator system according to Claim 1,
wherein the joints included in the second group include a redundant joint that has a redundant relationship with the joints included in the first group.

3. The medical manipulator system according to Claim 1 or 2,
wherein the control unit includes, as a control procedure of the first manipulator in the third mode,
a command stop step of stopping the control of the first manipulator based on the command,
a movement amount calculation step of calculating a movement amount of the first manipulator for canceling a contact state with the obstacle,
a movement step of moving the first manipulator by the movement amount calculated in the movement amount calculation step, and
a return step of shifting the third mode to a mode which is a mode before shift to the third mode and is one of the first mode and the second mode, and
wherein the control unit controls the first manipulator according to the control procedure of the first manipulator in the third mode.

4. The medical manipulator system according to Claim 1 or 2,
wherein the first manipulator further includes a second manipulation unit that is capable of manipulating the first manipulator and is different from the first manipulation unit,
wherein the control unit includes, as a control procedure of the first manipulator in the third mode,
a command stop step of stopping the control of the first manipulator based on the command, and
a permission step of permitting the second manipulation unit to manipulate the first manipulator, and
wherein the control unit controls the first manipulator according to the control procedure of the first manipulator in the third mode.

5. The medical manipulator system according to Claim 4,
wherein the second manipulation unit further includes an end detection unit that detects end of the manipulation performed by the second manipulation unit,
wherein the control unit further includes, as a control procedure of the first manipulator in the third mode, a return step of shifting the third mode to a mode which is a mode before shift to the third mode in a case in which the end detection unit detects the end of the manipulation performed by the second manipulation unit, the control unit controls the first manipulator according to the control procedure of the first manipulator in the third mode.
